# FASCICULE DE BREVET EUROPEEN

(11) **EP 1 893 135 B1**
(45) Date de publication et mention de la délivrance du brevet: **13.03.2013**
(21) Numéro de dépôt: 06794417.3
(22) Date de dépôt: 11.05.2006
(51) Int. Cl.: A61F 2/38

(54) **PROTHESE UNICOMPARTIMENTALE ANATOMIQUE DU GENOU**
UNIKONDYLÄRE ANATOMISCHE KNIEPROTHESE
UNICOMPARTMENTAL ANATOMIC KNEE PROSTHESIS

(30) Priorité: 13.05.2005 FR 0551253
(43) Date de publication de la demande: 05.03.2008
(73) Titulaire: Aubaniac, Jean-Manuel, 13100 Aix en Provence (FR)
(72) Inventeur: Aubaniac, Jean-Manuel, 13100 Aix en Provence (FR)
(74) Mandataire: Thivillier, Patrick
(86) Numéro de dépôt international: PCT/FR2006/050432
(87) Numéro de publication internationale: WO 2007/003809

(56) Documents cités:
- EP-A- 1 097 679
- DE-A1- 10 305 795
- US-A- 5 871 541
- US-A- 6 033 439
- US-A- 6 139 580

## Description

L'invention se rattache au secteur technique des implants orthopédiques notamment des prothèses unicompartimentales du genou.

D'une manière parfaitement connue pour un homme du métier, ce type de prothèse comprend un implant fémoral interne et/ou externe coopérant avec un implant tibial interne et/ou externe. Eventuellement, ce type de prothèse peut être complété par un implant fémoropatellaire.

Différentes solutions techniques ont été proposées.

On a également observé que, dans le domaine de la chirurgie unicompartimentale, il s'avère particulièrement intéressant d'atteindre les objectifs suivants :
- l'implant fémoral doit présenter, au niveau de sa partie postérieure, un profil anatomique déterminé afin de couvrir complètement la surface articulaire physiologique du condyle anatomique correspondant pour qu'en flexion complète à 165°, le contact avec le plateau tibial soit parfaitement harmonieux pour éviter l'usure du polyéthylène qui apparaît très souvent dans les prothèses unicompartimentales du genou, selon l'état de la technique.
- l'implant fémoral ne doit nécessiter, pour sa mise en place, que des découpes osseuses les plus économiques possibles ;
- le plateau tibial en polyéthylène doit savoir être mobile afin de permettre une translation antéropostérieure, médiolatérale et une rotation interne physiologique du tibia en flexion, pour harmoniser les rapports du couple de friction. Il en résulte une diminution de l'usure du polyéthylène. On observe que dans les prothèses unicompartimentales du genou selon l'état de la technique le plateau tibial interne ou externe est toujours fixé par différents moyens sur l'embase tibial sans aucune possibilité de mobilité ;
- l'ensemble de la prothèse doit pouvoir être implantée par une technique chirurgicale mini-invasive.

Pour atteindre ces différents objectifs, une solution avantageuse ressort de la demande de brevet WO02/09623 dont le demandeur de la présente est également titulaire. Les caractéristiques du préambule de la revendication 1 sont connues du document EP-A-1097679.

A partir des caractéristiques définies dans cette demande de brevet, on a voulu :
- améliorer, d'une manière significative, la fixation d'un implant condylien au niveau des condyles anatomiques correspondant interne ou externe ;
- améliorer les caractéristiques de mobilité du plateau tibial et unicompartimental par rapport à l'embase tibiale correspondante ;
- proposer un implant fémoropatellaire particulièrement adapté compte tenu des objectifs à atteindre, notamment dans le cas d'une technique chirurgicale mi-invasive.

Pour résoudre ces différents problèmes et atteindre ces objectifs, il a été conçu une prothèse unicompartimentale anatomique du genou selon les caractéristiques de la revendication 1.

Pour résoudre le problème posé d'assurer la fixation des implants fémoraux internes et/ou externes, à la partie correspondante du condyle anatomique considéré, une nervure faisant office d'ailette sagittale de positionnement et de fixation est réalisée entre la surface d'appui plane et courbe du patin condylien, ladite nervure étant apte à être engagée dans une fente formée dans l'épaisseur du condyle anatomique correspondant.

Avantageusement, la nervure présente, dans son épaisseur, des trous pour le passage de liens d'attache.

Des résultats techniques avantageux sont obtenus lorsque selon l'invention la surface d'appui plane de l'implant condylien fait un angle d'environ 50° par rapport à un plan horizontal.

Pour résoudre le problème posé d'assurer le guidage en rotation et en translation, du plateau tibial unicompartimental interne et/ou externe par rapport à l'embase correspondante, selon l'invention l'embase tibiale unicompartimentale présente un rebord rectiligne d'appui avec le massif des épines tibiales, ledit rebord étant asymétrique et présente, du côté interne, deux surfaces d'appui profilées aptes à coopérer avec le plateau tibial correspondant en fonction de son positionnement en interne ou en externe.

L'embase tibiale présente des agencements pour le montage avec capacité de déplacements limités et guidés en rotation et/ou en translation, du plateau tibial.

Avantageusement, pour résoudre le problème posé du guidage en rotation et en translation, dans une forme de réalisation, les agencements sont constitués par un puits médian formé à proximité du rebord d'appui de l'embase, la section longitudinale dudit puits étant elliptique ou oblongue, en étant apte à recevoir un plot cylindrique formé en débordement de la face de dessous du plateau tibial.

Selon une autre caractéristique et pour résoudre le problème posé de pratiquer des techniques chirurgicales mini-invasives, l'implant fémoropatellaire est profilé en section pour coopérer en appui avec l'échancrure anatomique intercondylienne et une partie des condyles anatomiques, et présente une partie apte à être insérée dans une fente formée au niveau de la tête fémorale.

L'invention est exposée ci-après plus en détail à l'aide des figures des dessins annexés dans lesquels :
- la figure 1 est une vue en perspective montrant un implant fémoro-tibial interne et externe dans le cas d'une prothèse unicompartimentale anatomique selon l'invention ;
- la figure 2 est une vue de face d'un implant condylien correspondant au condyle anatomique interne gauche ;
- la figure 3 est une vue de côté correspondant à la figure 2 ;
- la figure 4 montre la mise en place d'un implant condylien au niveau du condyle anatomique ;
- la figure 5 est une vue de dessus de l'embase tibiale unicompartimentale correspondant au compartiment interne gauche ;
- la figure 6 est une vue en coupe considérée selon la ligne 6-6 de la figure 5 ;
- la figure 7 est une vue en coupe considérée selon la ligne 7-7 de la figure 5 ;
- la figure 8 est une vue de dessus du plateau mobile interne ;
- la figure 9 est une vue en coupe considérée selon la ligne 9-9 de la figure 8 ;
- la figure 10 est une vue en coupe considérée selon la ligne 10-10 de la figure 8 ;
- la figure 11 est une vue de face d'un implant fémoropatellaire après mise en place au niveau de la tête fémorale ;
- la figure 12 est une vue en coupe transversale considérée selon la ligne 12-12 de la figure 11.

D'une manière connue et comme indiqué, la prothèse unicompartimentale anatomique du genou comprend un implant fémoral interne et/ou externe (F) coopérant en appui et à glissement avec un implant tibial interne et/ou externe (T).

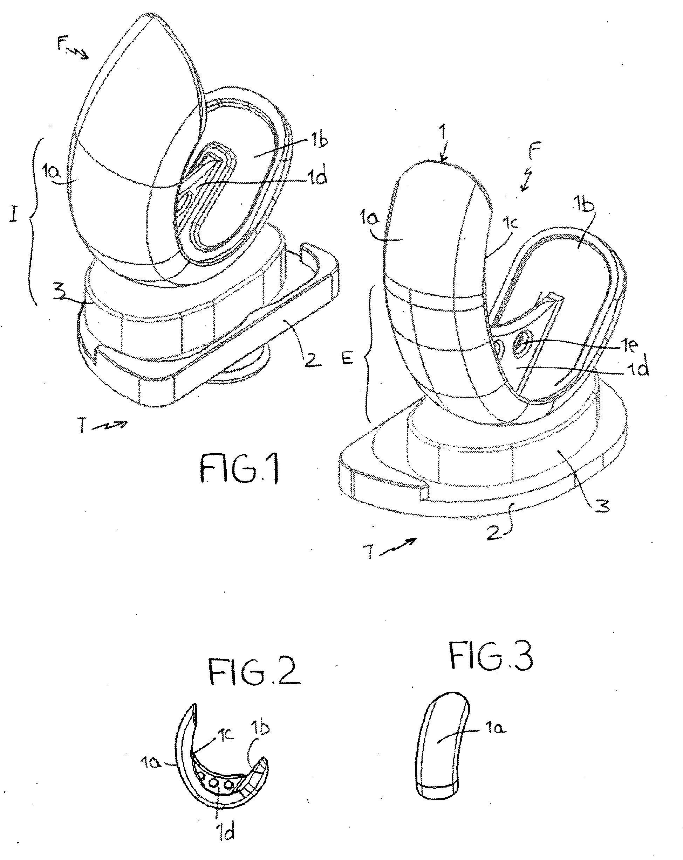

L'implant fémoral (F) est constitué par un patin condylien (1) présentant un profil externe (1a) du type anatomique, conformé pour largement envelopper le profil condyle anatomique correspondant. On renvoie aux figures 2 et 3 par exemple.

Selon l'invention, le patin condylien présente, du côté interne, une surface plane d'appui (1b) coopérant avec une coupe osseuse réalisée au niveau du bord postérieur du condyle. Cette surface d'appui plane (1b) fait un angle d'environ 50° par rapport à un plan horizontal. Cette surface plane (1b) est prolongée par une surface courbe d'appui (1c) apte à épouser le profil du bord antérieur du condyle anatomique correspondant.

Une nervure (1d) faisant office d'ailette de positionnement et de fixation, est réalisée entre les surfaces d'appui (1b) et (1c). Cette nervure (1d) est destinée à être engagée dans une fente formée dans l'épaisseur du condyle anatomique correspondant. Des trous (le) peuvent être formés dans l'épaisseur de l'ailette de fixation (1d) pour le passage de moyens d'attache sous forme de liens par exemple.

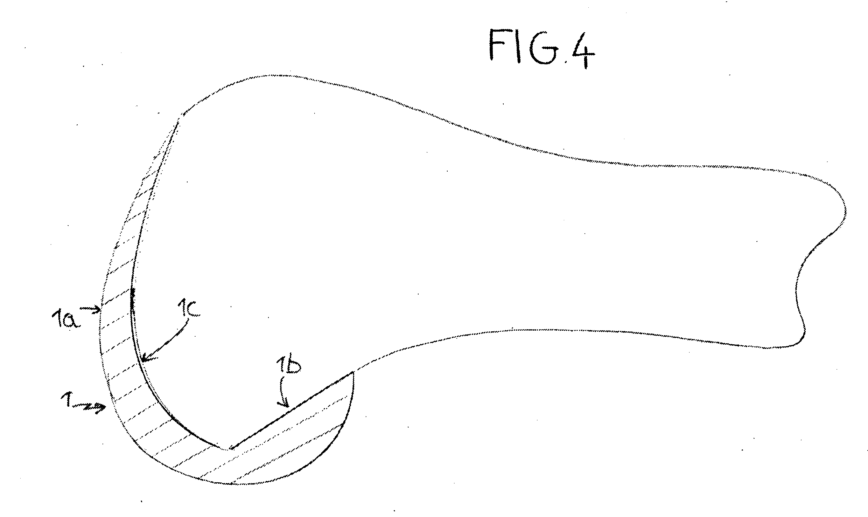

Comme le montre la figure 4, après impaction du patin condylien, ce dernier enveloppe parfaitement le profil externe du condyle anatomique correspondant, interne ou externe, droit ou gauche.

L'implant tibial comprend, du côté du compartiment interne tibial et/ou du compartiment externe tibial, une embase tibiale (2) recevant un plateau tibial (3) coopérant avec le patin condylien (1).

L'embase tibiale (2) du type unicompartimental présente un rebord rectiligne d'appui (2a) coopérant avec le massif des épines tibiales. Ce rebord est asymétrique et présente, du côté interne, deux surfaces d'appui profilées (2b) et (2c) aptes à coopérer avec le plateau tibial (3), notamment lorsque ce dernier est monté avec capacité de déplacement limité en rotation et en translation par rapport à ladite embase (2) comme indiqué dans la suite de la description. La fixation de l'embase (2), au niveau de la coupe tibiale réalisée d'une manière parfaitement connue pour un homme du métier, s'effectue par tout moyen connu et approprié.

D'une manière avantageuse, l'embase tibiale (2) présente des agencements pour le montage avec capacité de déplacement limité et guidé en rotation et/ou en translation du plateau tibial (3).

Dans ce but, un puits médian (2d) est formé à proximité du rebord d'appui (2a). La section transversale du puits (2d) est elliptique (figures des dessins) ou oblongue. Ce puits (2d) est destiné à recevoir un plot cylindrique (3a) formé en débordement de la face de dessous du plateau tibial (3). Compte tenu de ces dispositions, il en résulte que les mouvements de rotation et/ou de translation du plateau mobile (3) par rapport à l'embase (2), sont limités et préétablis avec des effets de butée, d'une part au niveau du puits (2d) et, d'autre part, au niveau des rebords d'appui (2b) ou (2c).

Sans pour cela sortir du cadre de l'invention, on n'exclut pas de monter le plateau (3) d'une manière fixe par rapport à l'embase tibiale (2).

L'ensemble de l'embase unicompartimentale tibiale (2) est profilée en fonction du compartiment tibial interne ou en fonction du compartiment tibial externe.

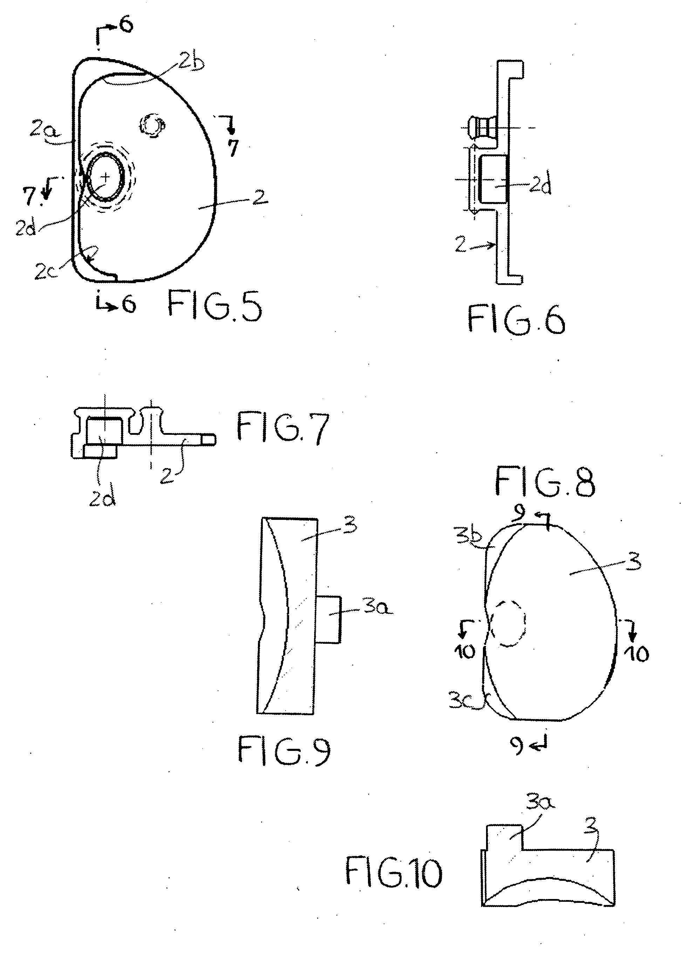

A noter également que, dans le cas d'un plateau mobile (3), ce dernier présente, vue en plan, une forme générale elliptique (figure 8) avec, du côté du massif des épines tibiales, deux pans coupés symétriques et arrondis (3b) et (3c).

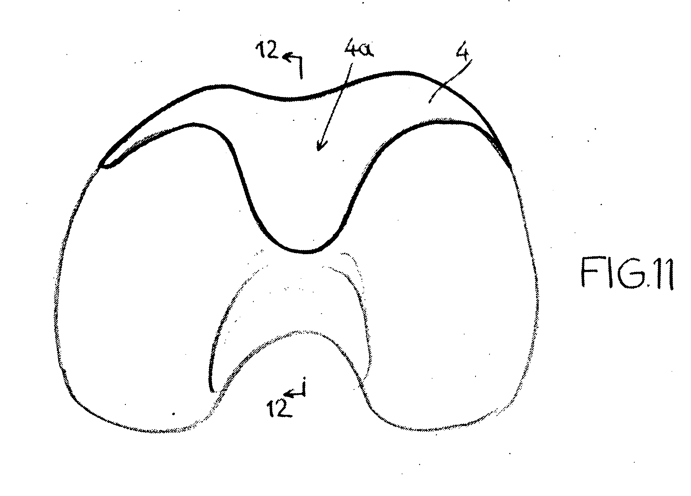

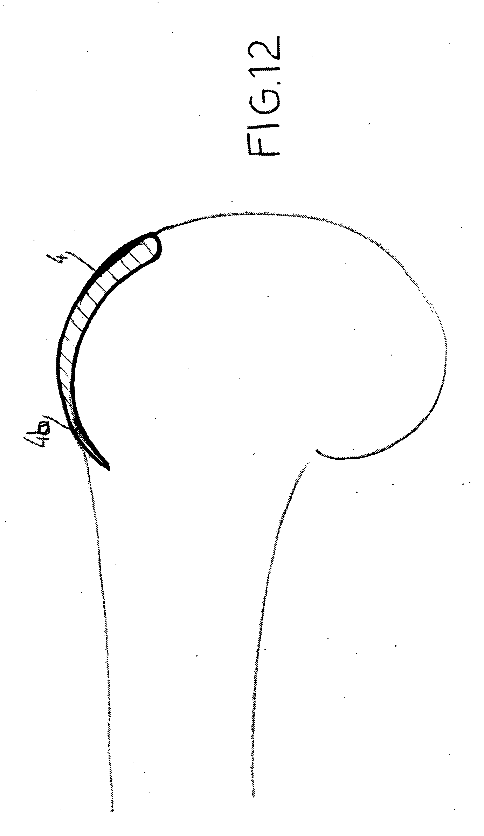

Selon une autre caractéristique, la prothèse unicompartimentale anatomique du genou selon l'invention, comprend un implant fémoropatellaire (4) (figures 11 et 12). Cet implant (4) présente une surface profilée de glissement (4a) conformée en section pour coopérer en appui avec l'échancrure anatomique intercondylienne et une partie du condyle anatomique. Cette surface de glissement (4a) coopère avec un bouton rotulien non représenté.

D'une manière avantageuse, la surface de glissement (4a) présente une partie (4b) apte à être insérée dans une fente formée au niveau de la tête fémorale (figure 12).

A titre indicatif nullement limitatif, le patin condylien et les embases tibiales peuvent être réalisés en toute matière métallique du type de celle couramment utilisée dans le domaine de l'orthopédie, tandis que les plateaux tibiaux (3) peuvent être réalisés en polyéthylène.

Selon l'invention, en considérant les caractéristiques spécifiques des patins condyliens, des embases tibiales, des plateaux tibiaux et, compte tenu du but recherché de respecter l'anatomie, la prothèse comprend :
- un patin condylien interne anatomique (droit et gauche) ;
- un patin condylien externe anatomique (droit et gauche), chacun de ces patins ayant par conséquent des formes tout à fait différentes correspondant à l'anatomie ;
- une embase tibiale interne anatomique (droite et gauche) avec un plateau mobile interne à déplacement déterminé ;
- une embase tibiale externe anatomique (droite et gauche) avec un plateau mobile externe à déplacement optimisé.

On renvoie à la figure 1 qui montre les différents composants prothétiques pour le compartiment interne (I) et le compartiment externe (E) dans le cas d'un genou considéré du côté gauche.

On observe également une différence au niveau de la forme elliptique du puits de l'embase interne et externe considérée, créant une différence de déplacement des plateaux tibiaux correspondants.

Les avantages ressortent bien de la description, en particulier on souligne et on rappelle la technique de pose mini-invasive avec un minimum de coupes à réaliser résultant des caractéristiques spécifiques du profil des implants.

## Revendications

1. Prothèse unicompartimentale anatomique du genou comprenant un implant fémoral interne et/ou externe (F) coopérant avec un implant tibial interne et/ou externe (T), en combinaison ou non avec un implant fémoropatellaire (4), l'implant fémoral étant constitué par un patin condylien (1) présentant un profile externe (1a) du type anatomique, et implant tibial étant constitué par un plateau (3) monté avec capacité de déplacements limités en rotation et en translation dans un plan horizontal sur une embase tibiale (2) figée au niveau d'une coupe osseuse réalisée dans le compartiment tibial anatomique correspondant **caractérisée en ce que**
- l'implant fémoral présente, du côté interne, une surface plane d'appui (1b) qui fait fait un angle d'environ 50° par rapport à un plan horizontal, en étant apte à coopére avec une coupe osseuse réalisée au niveau du bord postérieur du condyle anatomique de manière à envelopper ledit bord, ladite surface plane (1b) étant prolongée par une surface courbe d'appui (1c) apte à épouser le profil du bord antérieur du condyle anatomique ;
- l'embase tibiale unicompartimentale (2) présente un rebord rectiligne (2a) d'appui avec le massif des épines tibiales, ledit rebord étant asymétrique et présentant, du côté interne, deux surfaces d'appui profilées (2b) et (2c) aptes à coopérer avec le plateau tibial correspondant (3) en fonction de son positionnement en interne ou en externe.

2. Prothèse selon la revendication 1, **caractérisée en ce qu'**une nervure (1d) faisant office d'ailette sagittale de positionnement et de fixation est réalisée entre la surface d'appui plane (1b) et courbe (1c) du patin condylien (1), ladite nervure (1d) étant apte à être engagée dans une fente formée dans l'épaisseur du condyle anatomique correspondant.

3. Prothèse selon la revendication 2, **caractérisée en ce que** la nervure (1d) présente, dans son épaisseur, des trous pour le passage de liens d'attache.

4. Prothèse selon l'une quelconque des revendications 1 et 3, **caractérisée en ce que** l'embase tibiale (2) présente des agencements pour le montage avec capacité de déplacements limités et guidés en rotation et/ou en translation, du plateau tibial (3).

5. Prothèse selon la revendication 4, **caractérisée en ce que** les agencements sont constitués par un puits médian (2d) formé à proximité du rebord d'appui de l'embase (2), la section longitudinale dudit puits étant elliptique ou oblongue, en étant apte à recevoir un plot cylindrique (3a) formé en débordement de la face de dessous du plateau tibial (3).

6. Prothèse selon la revendication 1, **caractérisée en ce que** l'implant fémoropatellaire (4) est profilé en section pour coopérer en appui avec l'échancrure anatomique intercondylienne et une partie des condyles anatomiques, et présente une partie apte à être insérée dans une fente formée au niveau de la tête fémorale.

7. Prothèse selon l'une quelconque des revendications 1 à 6, **caractérisée en ce qu'**elle comprend :
- un patin condylien interne de forme anatomique (droit et gauche) ;
- un patin condylien externe de forme anatomique (droit et gauche) ;
- une embase tibiale interne de forme anatomique (droite et gauche) coopérant avec un plateau tibial mobile interne à déplacement déterminé;
- une embase tibiale externe de forme anatomique (droite et gauche) coopérant avec un plateau tibial mobile externe à déplacement optimisé.

## Claims

1. Anatomical unicompartmental knee prosthesis comprised of a medial and/or lateral femoral implant (F) interacting with a medial and/or lateral tibial implant (T), combined or not with a femoropatellar implant (4), the femoral implant consisting of a condylar component (1) with an anatomical external profile (1a), and the tibial implant consisting of a plateau (3) mounted, in such a way that its displacement in rotation and translation in a horizontal plane is limited, on a tibial baseplate (2) fixed at the site of bone resection in the corresponding anatomical tibial compartment,
**characterized by**
- the femoral implant having, on the inside, a flat weight-bearing surface (1b) making an angle of about 50° with a horizontal plane, that is able to interact with an area of resected bone on the posterior edge of the anatomical condyle such that it envelopes the said edge, the said flat surface (1b) being prolonged by a curved weight-bearing surface (1c) that marries the anterior edge of the anatomical condyle;
- the unicompartmental tibial baseplate (2) having a rectilinear weight-bearing border (2a) supported by the intercondylar eminences, the said border being asymmetrical, having on the inside two profiled weight-bearing surfaces (2b) and (2c) able to interact with the corresponding tibial plateau (3) depending on its position medially and laterally.

2. Prosthesis according to claim 1, **characterized by** its having a rib (1d) acting as a sagittal positioning and fixing fin between the flat and curved weight-bearing surfaces (1b) and (1c) of the condylar component (1), the said rib (1d) being able to engage in a slot created in the thickness of the corresponding anatomical condyle.

3. Prosthesis according to claim 2, **characterized by** holes having been made in the thickness of the rib (1d) for passing through means of attachment.

4. Prosthesis according to either of claims 1 and 3, **characterized by** the tibial baseplate (2) having an assembly system producing limited and guided displacement, in rotation and/or translation, of the tibial plateau (3).

5. Prosthesis according to claim 4 **characterized by** the system being composed of a median well (2d) created near the weight-bearing border of the baseplate (2), the longitudinal section of the said well being elliptical or oblong, and suitable for receiving a cylindrical lug (3a) projecting from the underside of the tibial plateau (3).

6. Prosthesis according to claim 1, **characterized by** the femoropatellar implant (4) having a sectional profile for supportive interaction with the anatomical intercondylar hollow and part of the anatomical condyles, and having a part that can be inserted in a slot created in the head of the femur.

7. Prosthesis according to any one of the claims 1 to 6, **characterized by** its comprising:
- an anatomically shaped medial condylar component (right and left);
- an anatomically shaped lateral condylar component (right and left);
- an anatomically shaped medial tibial baseplate (right and left) interacting with a medial mobile tibial plateau with specific displacement capacity;
- an anatomically shaped lateral tibial baseplate (right and left) interacting with a lateral mobile tibial plateau with optimized displacement capacity.

## Patentansprüche

1. Anatomische Unikompartimentprothese des Knies mit einem inneren und/oder äußeren Femurimplantat (F) das - in Verbindung mit einem femurpatellaren Implantat (4) oder nicht - mit einem inneren und/oder äußeren Tibiaimplantat (T) zusammenwirkt, wobei das Femurimplantat aus einem Kondylenschuh (1) mit einem anatomischen Außenprofil (1a) und das Tibiaimplantat aus einer Platte (3) besteht, die mit begrenzter Beweglichkeit in Rotation und Translation in einer horizontalen Ebene auf einer Tibiabasis (2), die im Bereich eines Knochenschnitts im entsprechenden anatomischen Tibiakompartiment fixiert ist, angebracht ist,
**dadurch gekennzeichnet, dass**
- das Femurimplantat auf der Innenseite eine ebene Auflagefläche (1b) aufweist, die gegenüber einer horizontalen Ebene einen Winkel von ca. 50° beschreibt und geeignet ist, mit einem Knochenschnitt im Bereich des hinteren Randes des anatomischen Kondylus zusammenzuwirken, um den besagten Rand zu umhüllen, wobei die besagte ebene Fläche (1b) durch eine gekrümmte Auflagefläche (1c) verlängert wird, die geeignet ist, sich an das Profil des vorderen Randes des anatomischen Kondylus anzupassen;
- die unikompartimentale Tibiabasis (2) einen geradlinigen, auf den Eminentia intercondylaris aufliegenden Rand (2a) aufweist, wobei der besagte Rand asymmetrisch ist und auf der Innenseite zwei profilierte Auflageflächen (2b und 2c) aufweist, die geeignet sind, abhängig von der innerer oder äußerer Positionierung mit dem entsprechenden Tibiaplateau (3) zusammenzuwirken.

2. Prothese nach Anspruch 1, **dadurch gekennzeichnet, dass** zwischen der ebenen (1b) und gekrümmten (1c) Auflagefläche des Kondylenschuhs (1) ein Steg (1d) ausgebildet ist, der als sagittale Positionier- und Befestigungsrippe dient, wobei der besagte Steg (1d) geeignet ist, in eine Spalte in der Materialdicke des entsprechenden anatomischen Kondylus eingeführt zu werden.

3. Prothese nach Anspruch 2, **dadurch gekennzeichnet, dass** der Steg (1d) in seiner Materialdicke Löcher für die Durchführung von Befestigungsverbindungen aufweist.

4. Prothese nach Anspruch 1 bzw. 3, **dadurch gekennzeichnet, dass** die Tibiabasis (2) Vorkehrungen zum Anbringen des Tibiaplateaus (3) mit begrenzter und geführter Beweglichkeit in Rotation und/oder Translation aufweist.

5. Prothese nach Anspruch 4, **dadurch gekennzeichnet, dass** die Vorkehrungen aus einer mittleren Einsenkung (2d) bestehen, die in der Nähe des Auflagerandes der Tibiabasis (2) ausgebildet ist, wobei der Längsschnitt der besagten Einsenkung elliptisch oder länglich ist und geeignet ist, einen zylindrischen Klotz (3a) aufzunehmen, der aus der Unterseite des Tibiaplateaus (3) vorspringend ausgeformt ist.

6. Prothese nach Anspruch 1, **dadurch gekennzeichnet, dass** das femurpatellare Implantat (4) im Querschnitt so ausgebildet ist, um in Auflage mit der anatomischen interkondylären Aussparung und einem Teil der anatomischen Kondylen zusammenzuwirken, und einen Teil aufweist, der geeignet ist, in einen Spalt im Bereich des Femurkopfs eingeführt zu werden.

7. Prothese nach einem der Ansprüche 1-6, **dadurch gekennzeichnet, dass** sie die nachstehenden Bestandteile umfasst:
- einen inneren Kondylenschuh mit anatomischer Form (rechts und links);
- einen äußeren Kondylenschuh mit anatomischer Form (rechts und links);
- eine innere Tibiabasis mit anatomischer Form (rechts und links), die mit einem mobilen inneren Tibiaplateau mit vorgegebener Beweglichkeit zusammenwirkt;
- eine äußere Tibiabasis mit anatomischer Form (rechts und links), die mit einem äußeren mobilen Tibiaplateau mit optimierter Beweglichkeit zusammenwirkt.
